# EUROPÄISCHE PATENTSCHRIFT

(11) **EP 0 788 767 B1**
(45) Veröffentlichungstag und Bekanntmachung des Hinweises auf die Patenterteilung: **16.04.2003**
(21) Anmeldenummer: 97200210.9
(22) Anmeldetag: 27.01.1997
(51) Int. Cl.: A61B 6/04

(54) **Röntgendiagnostikgerät**
X-ray diagnostic apparatus
Appareil diagnostique à rayons X

(30) Priorität: 06.02.1996 DE 19605627
(43) Veröffentlichungstag der Anmeldung: 13.08.1997
(73) Patentinhaber: Philips Corporate Intellectual Property GmbH, 20099 Hamburg (DE); Koninklijke Philips Electronics N.V., 5621 BA Eindhoven (NL)
(72) Erfinder: Csikos, Janos, Dr., Röntgenstrasse 24, 22335 Hamburg (DE); Medgyesi, György, Röntgenstrasse 24, 22335 Hamburg (DE)
(74) Vertreter: Volmer, Georg, Dipl.-Ing.

(56) Entgegenhaltungen:
- EP-A- 0 125 713
- FR-A- 1 273 267
- GB-A- 845 783
- HU-B- 207 431

## Beschreibung

Die Erfindung betrifft ein Röntgendiagnostikgerät mit einem Tischgestell und mit einer Basiseinheit zur Lagerung des Tischgestells, welches um eine horizontale Achse schwenkbar ist, und mit einer Verschiebevorrichtung zur Verschiebung des Tischgestell gegenüber der Basiseinheit.

Ein derartiges Röntgendiagnostikgerät ist aus der HU-PS 207 431 bekannt. Die Verschiebung des Tischgestells gegenüber der Basiseinheit wird dort durch einen Spindelantrieb bewirkt. An dem Tischgestell ist eine um ihre Längsachse drehbare Spindel angebracht. Diese Spindel wird über eine Kette von einem am Tischgestell angebrachten Motor angetrieben. Auf der Spindel sitzt eine Gewindemutter, die zusammen mit einem Gleitklotz an der mit dem Boden verbundenen Basiseinheit angebracht ist. Der Gleitklotz gleitet in einer parallel zu der Spindel am Tischgestell angebrachten Führungsschiene. Wenn die Spindel angetrieben wird, wird das Tischgestell gegenüber der Basiseinheit verschoben. An einer weiteren Stelle an der Basiseinheit ist eine Führungsrolle angebracht, die in einer am Tischgestell angebrachten Führungsschiene abrollt. Aufgrund der Krümmung dieser Führungsschiene wird gleichzeitig mit der Verschiebung dem Tischgestell eine Schwenkbewegung um eine horizontale Achse aufgezwungen. Der Gleitklotz mit der Gewindemutter ist dazu derartig an der Basiseinheit gelagert, daß er dieser Schwenkbewegung um die horizontale Achse folgen kann.

Die Herstellung der bei diesem Röntgendiagnostikgerät verwendeten gekrümmten Führungsschiene ist sehr aufwendig und teuer, da sehr enge Fertigungstoleranzen eingehalten werden müssen, um die gewünschte Schwenkbewegung zu bewirken. Ein weiteres Problem ist die Lagerung des Gleitklotzes an der Basiseinheit. Auf dieser lastet dauerhaft der größte Teil des Gewichts, weshalb der Verschleiß dieser Lager sehr hoch ist. Insgesamt ist dieser Aufbau des Antriebs kompliziert und erfordert eine Vielzahl von fertigungstechnisch sehr präzise und deshalb teuer herzustellenden Bauteilen.

Der Erfindung liegt deshalb die Aufgabe zugrunde, ein Röntgendiagnostikgerät der eingangs genannten Art derart auszugestalten, daß mit einfachen, möglichst verschleißfreien und kostengünstigen Bauteilen die gewünschte Schwenkbewegung möglich ist.

Diese Aufgabe wird erfindungsgemäß dadurch gelöst, daß an einem Gelenkpunkt an der Basiseinheit ein erster Punkt einer Führungsstange angebracht ist und daß an einem Gelenkpunkt am Tischgestell ein zweiter Punkt der Führungsstange angebracht ist, so daß bei der Verschiebung des Tischgestells gegenüber der Basiseinheit eine Schwenkbewegung des Tischgestells erzwungen wird.

Die Schwenkbewegung des Tischgestells wird hier durch deutlich einfachere Bauteile bewirkt. Die Führungsstange ist eine einfache, bevorzugt gerade ausgebildete Stange, die Zug- und Druckkräft aufnehmen muß. Die Führungsstange ist gelenkig derart an der Basiseinheit bzw. am Tischgestell angebracht, daß sie eine Schwenkbewegung in einer Ebene machen kann, auf der die horizontale Schwenkachse des Tischgestells senkrecht steht. Eine derartige gelenkige Befestigung ist beispielsweise durch einfache Kugellager möglich.

Mit dem erfindungsgemäßen Röntgendiagnostikgerät ist eine Schwenkbewegung des Tischgestells um mindestens 120° möglich, also von einer Position mit senkrecht stehendem Tisch (Fußtieflage) bis in eine Position, bei der der Tisch um mindestens 30° in die andere Richtung gegenüber der horizontalen Lage verkippt ist (Kopftieflage). Dadurch sind Röntgenaufnahmen eines Patienten in verschiedenen Positionen möglich. Die Schwenkung des Tischgestells erfolgt gleichmäßig und ruhig ohne ruckartige Bewegungen.

In einer Ausgestaltung ist erfindungsgemäß vorgesehen, daß das Tischgestell eine Führungsschiene aufweist, die auf einem an der Basiseinheit gelenkig angebrachten Gleitklotz gleitet. Ein großer Teil des Gewichts des Geräts lastet auf der Verschiebevorrichtung. Um diese zu entlasten und damit den Verschleiß an der Verschiebevorrichtung möglichst gering zu halten, wird bei dieser Ausgestaltung ein großer Teil des Gewichts vom Gleitklotz aufgenommen. Der Gleitklotz ist an der Basiseinheit derart gelagert, daß er der Schwenkbewegung des Tischgestells folgen kann, d.h., daß er synchron zum Tischgestell um seine Lagerachse geschwenkt wird.

Um das Gewicht des Geräts noch mehr zu verteilen, ist in einer weiteren Ausgestaltung erfindungsgemäß vorgesehen, daß das Tischgestell zwei parallele Führungsschienen und die Basiseinheit zwei gelenkig angebrachte Gleitklötze aufweist, auf denen jeweils eine der Führungsschienen gleitet. Dadurch wird zusätzlich eine bessere Abstützung des Tischgestells gewährleistet und ein Kippen des Tischgestells um eine horizontale, senkrecht zur horizontalen Schwenkachse verlaufenden Längsachse des Tischgestells verhindert.

Vorteilhaft ist die Erfindung derart ausgestaltet, daß zwei parallele Führungsstangen vorgesehen sind und daß die Gelenkpunkte an der Basiseinheit bzw. am Tischgestell jeweils auf einer gemeinsamen horizontalen Achse liegen. Dies trägt dazu bei, die Stabilität des Geräts während der Schwenkbewegung zu erhöhen. Der Verschleiß wird zu dem aufgrund der Verteilung der Kräfte auf möglichst viele Bauteile geringer gehalten.

Bevorzugt ist in einer erfindungsgemäßen Ausgestaltung vorgesehen, daß die Verschiebevorrichtung eine am Tischgestell angebrachte Zahnstange und ein an der Basiseinheit drehbar gelagertes, in die Zahnstange greifendes Zahnrad umfaßt. Diese Bauteile sind sehr einfach und kostengünstig herzustellen. Der Verschleiß ist außerdem an derartigen Bauteilen sehr gering. Selbst ein Auswechseln derartig einfacher Bauteile ist sehr leicht möglich.

Besonders vorheilhaft sieht eine erfindungsgemäße Weiterbildung vor, daß die Verschiebevorrichtung zwei parallele, am Tischgestell angebrachte Zahnstangen und zwei parallele, an der Basiseinheit drehbar gelagerte, in jeweils eine Zahnstange greifende Zahnräder umfaßt, welche auf einer gemeinsamen, von einem Motor angetriebenen Antriebswelle befestigt sind. Dies bewirkt, daß die Schwenkbewegung besonders ruhig und gleichmäßig erfolgt.

In einer Weiterbildung der Erfindung ist alternativ dazu vorgesehen, daß die Verschiebevorrichtung eine am Tischgestell angebrachte Spindel und eine an der Basiseinheit drehbar gelagerte, in die Spindel greifende Gewindemutter umfaßt. Auch durch diese relativ einfache Ausgestaltung wird die gewünschte Verschiebung des Tischgestells gegenüber der Basiseinheit erreicht.

Eine besonders einfache und kostengünstige Herstellung ergibt sich bei gerade ausgebildeten Bauteilen. So sind beispielsweise die Führungsschiene und die Zahnstange gerade ausgebildet und parallel zueinander angeordnet.

Die Erfindung wird nachfolgend anhand der Zeichnung näher erläutert. Es zeigen:
- Fig. 1: ein erfindungsgemäßes Röntgendiagnostikgerät in einer Seitenansicht,
- Fig. 2: das Röntgendiagnostikgerät gemäß Fig. 1 in einer Vorderansicht,
- Fig. 3: eine Detaildarstellung aus Fig. 2,
- Fig. 4 und 5: das Röntgendiagnostikgerät gemäß Fig. 1 in verschiedenen Schwenkpositionen und
- Fig. 6: eine weitere Ausführung eines erfindungsgemäßen Röntgendiagnostikgerätes mit einem Spindelantrieb.

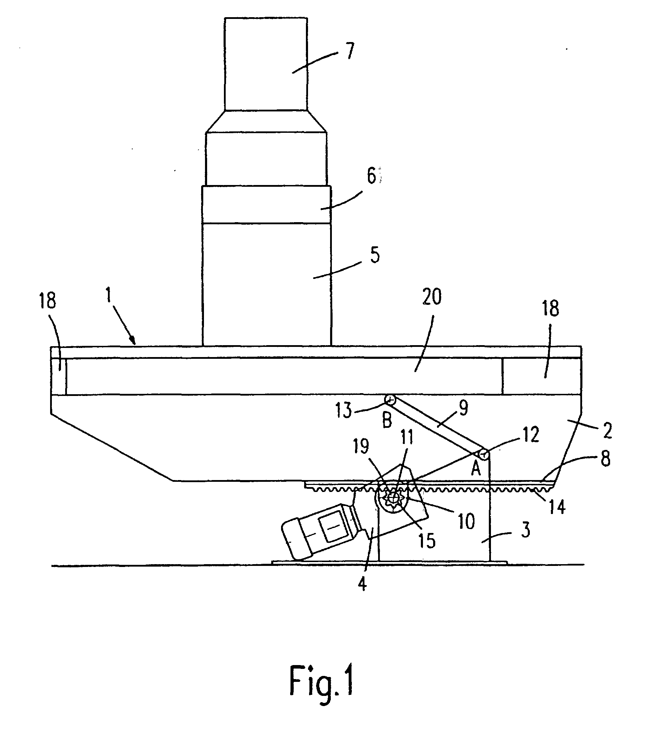

Das in Fig. 1 dargestellte Röntgendiagnostikgerät weist eine Tischplatteneinheit 18 mit einer Tischplatte 1 auf, die auf einem Tischgestell 2 mittels einer Laufrastereinheit 20 verschiebbar gelagert ist. Unterhalb der Tischplatte 1 befindet sich eine nicht dargestellte Röntgenröhre, die an einem Träger 5 befestigt ist. Oberhalb der Tischplatte 1 ist an dem Träger 5 ein Röntgenzielgerät 6 mit einem Bildverstärker 7 befestigt. Unterhalb der Tischplatte 1 befindet sich eine mit dem Boden verbundene Basiseinheit 3 sowie ein Motor 4. Senkrecht zur Zeichenebene ist eine Antriebswelle 19 angeordnet, deren Mittelachse die Achse 11 bildet. Diese Antriebswelle 19 ist in der Basiseinheit 3 gelagert und wird von dem Motor 4 angetrieben. Auf der Antriebswelle 19 sitzen zwei Zahnräder 15. An der Unterseite des Tischgestells 2 sind zwei parallele, parallel zur Tischplatte 1 verlaufende, gerade ausgebildete Zahnstangen 14 angeordnet. In jeweils eine dieser Zahnstangen 14 greift eines der Zahnräder 15.

Auf der Antriebswelle 19 sind außerdem zwei Gleitklötze 10 gelagert. Auf jeder dieser Gleitklötze 10 gleitet eine, an der Unterseite des Tischgestells 2 parallel zu den Zahnstangen 14 verlaufende Führungsschiene 8.

In der Basiseinheit 3 sind auf einer zur Achse 11 parallelen Achse 12 die Enden zweier paralleler Führungsstangen 9 in den Gelenkpunkten A gelagert. Das andere Ende der beiden Führungsstangen 9 ist auf einer zur Achse 12 parallelen weiteren Achse 13 am Tischgestell 2 in den Gelenkpunkten B gelagert, so daß die Führungsstangen 9 in zur Zeichenebene parallelen Ebenen um die Achse 12 schwenkbar sind.

Soll die Tischplatte 1 aus der gezeigten horizontalen Lage in eine andere, beispielsweise in die vertikale Lage gebracht werden, so wird die Antriebswelle 19 mit den zwei Zahnrädern 15 von dem Motor 4 angetrieben. Die Zahnräder 15 greifen in die Zahnstangen 14, und dadurch wird das Tischgestell 2 gegenüber der Basiseinheit 3 verschoben. Das Tischgestell 2 gleitet dabei mit den Führungsschienen 8 auf den Gleitklötzen 10. Gleichzeitig bewirken bei dieser Verschiebung die Führungsstangen 9, daß das Tischgestell 2 um eine zur Achse 11 parallele, im Raum veränderliche horizontale Achse geschwenkt wird. Der Gelenkpunkt A einer Führungsstange 9 bewegt sich bei der Schwenkbewegung des Tischgestells 2 auf einem Kreisbogen um den Gelenkpunkt B dieser Führungsstange in einer zur Zeichenebene der Fig. 1 parallelen Ebene.

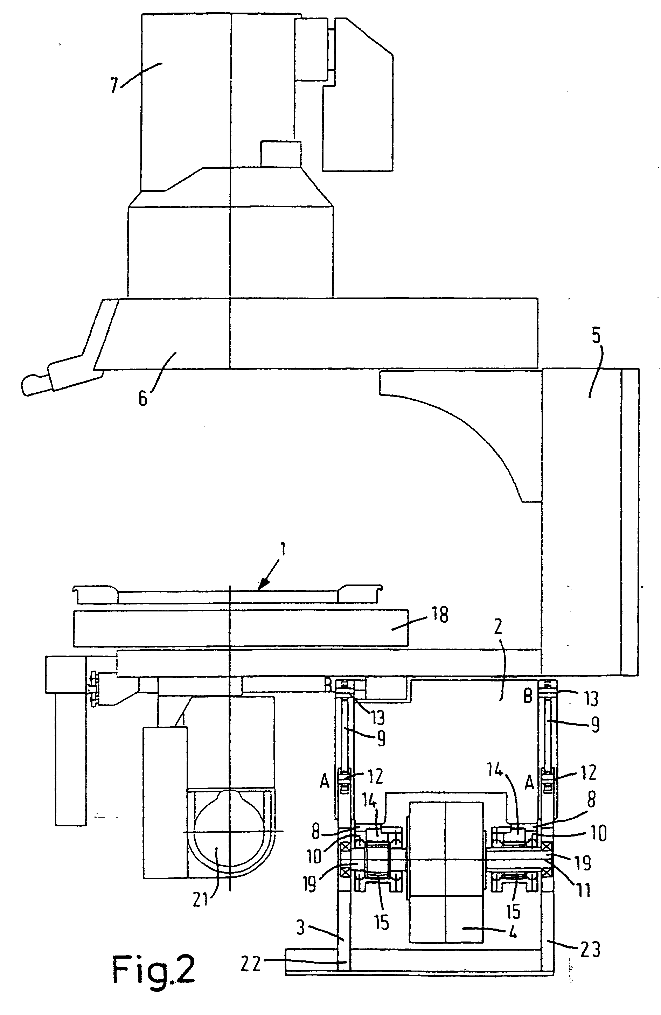

In Fig. 2 ist das Röntgendiagnostikgerät gemäß Fig. 1 in einer Vorderansicht dargestellt. Zu erkennen ist hier eine Röntgenröhre 21 unterhalb der Tischplatte 1. Daneben befindet sich das Tischgestell 2 und darunter die Basiseinheit 3. Die Basiseinheit 3 weist dabei zwei senkrecht stehende Wände 22 und 23, beispielsweise aus Metall auf, in denen die Antriebswelle 19 gelagert ist. Auf der Antriebswelle 19 sitzt zu beiden Seiten des Motors 4 jeweils ein Zahnrad 15. Außerdem ist auf der Antriebswelle 19 zu beiden Seiten des Motors 4 jeweils ein Gleitklotz 10 angeordnet. Die Gleitklötze 10 sind auf der Antriebswelle 19 gelagert, d.h., sie sind nicht fest mit der Antriebswelle 19 verbunden und folgen nicht der Antriebsdrehbewegung der Antriebswelle 19.

An dem Tischgestell 2 ist oberhalb der Gleitklötze 10 jeweils eine Führungsschiene 8 angebracht. Teilweise neben und teilweise unterhalb jeder Führungsschiene 8 ist eine Zahnstange 14 fest am Tischgestell 2 angebracht. Oberhalb der Achse 11 verläuft durch die beiden Wände 22 und 23 die Achse 12 mit den Gelenkpunkten A der Führungsstangen 9. Oberhalb der Achse 12 verläuft die Achse 13, auf der sich die Gelenkpunkte B der Führungsstangen 9 befinden.

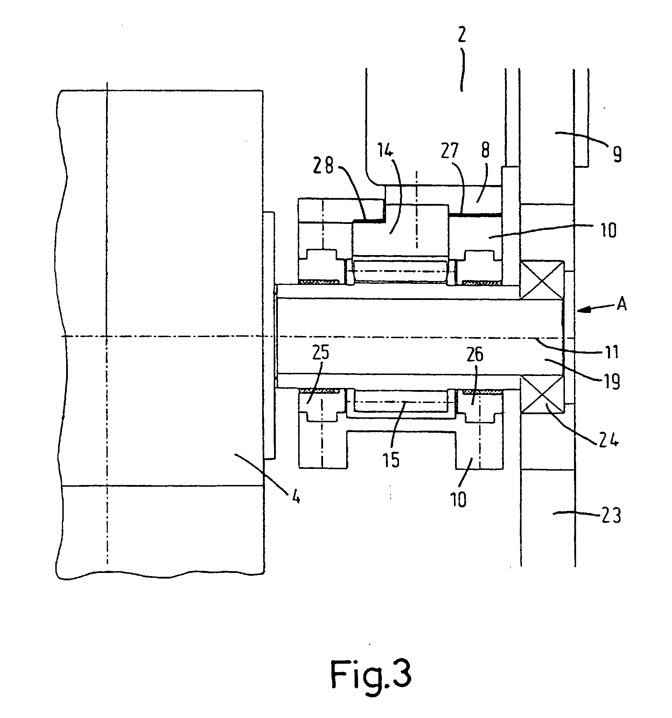

Anhand der Detaildarstellung in Fig. 3 soll insbesondere die Ausgestaltung des Gleitkotzes 10 näher erläutert werden. Dargestellt ist der in Fig. 2 rechts des Motors 4 angeordnete Bereich um die Antriebswelle 19. Die im folgenden beschriebenen Elemente sind in einer praktischen Ausgestaltung vorteilhafterweise symmetrisch zur Mittelachse des Motors 4 auch auf der linken Seite des Motors 4. angeordnet, der Übersichtlichkeit halber jedoch weggelassen. Für die Funktion der Verschiebevorrichtung würden auch bereits die gezeigten, einseitig des Motors 4 angeordneten Elemente ausreichen.

Die Antriebswelle 19 ist in der Wand 23 der Basiseinheit 3 mittels eines Lagers 24 gelagert. Das fest auf der Antriebswelle 19 sitzende Zahnrad 15 greift in die Zahnstange 14. Die Zahnstange 14 ist fest mit einem Teil der Führungsschiene 8 verbunden, welche wiederum fest an dem Tischgestell 2 angebracht ist. Zu beiden Seiten des Zahnrades 15 sitzt jeweils ein Lager 25 und 26, auf denen der Gleitklotz 10 gelagert ist. Auf dem Gleitklotz 10 gleitet die Führungsschiene 8 auf der Gleitfläche 27.

Auf der zum Motor 4 hin liegenden Seite weist die Zahnstange 14 an der Oberseite eine Aussparung auf, in die ein Teil des Gleitklotzes 10 greift, so daß sich zwischen Gleitklotz 10 und Zahnstange 14 eine Gleitfläche 28 ergibt.

Der Gleitklotz 10 und die Führungsschiene 8 dienen dazu, einen großen Teil des Gewichts des Geräts und des Patienten aufzunehmen. Eine Ausgestaltung des Röntgendiagnostikgeräts ohne Führungsschiene 8 und Gleitklotz 10 ist auch möglich, würde jedoch zu einem großen Verschleiß der Zahnstange 14 und des Zahnrades 15 führen, da diese beiden Elemente dann einen Großteil des Gewichts tragen müßten. Bei der gezeigten Ausführungsform trägt der Gleitklotz 10 auf der Gleitfläche 27 den größten Teil des Gewichts. Ein kleiner Teil des Gewichts lastet je nach Schwenklage des Tischgestells 2 auch auf der Gleitfläche 28.

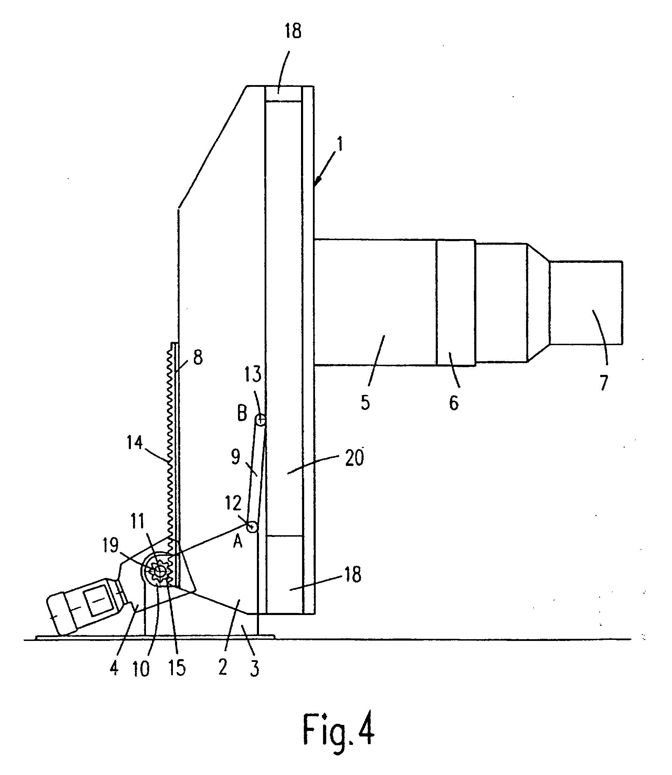

In Fig. 4 ist das erfindungsgemäße Röntgendiagnostikgerät bei senkrechter Lage der Tischplatte 1 (Fußtieflage) dargestellt. Das Zahnrad 15 hat das Tischgestell 2 mit der Zahnstange 14 derart verschoben, daß das Zahnrad 15 an einem Ende der Zahnstange 14 angelangt ist. Die Führungsstange 9 hat dabei die Schwenkung des Tischgestells 2 und der Tischplatte 1 in die senkrechte Position erzwungen. In der gezeigten Position muß die Führungsstange 9 fast das gesamte Gewichts aufnehmen. Eine geringe Kraft wirkt auf den Gleitklotz 10 und belastet hier die Gleitfläche 28 (siehe Fig. 3).

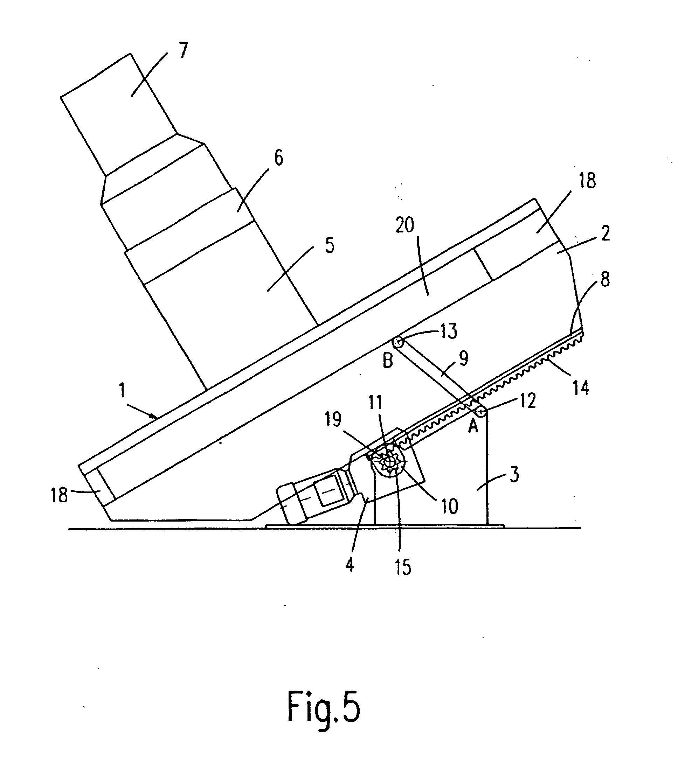

In Fig. 5 ist das erfindungsgemäße Röntgendiagnostikgerät in einer Position gezeigt, bei der der Tisch 1 um 30° zur horizontalen Lage in die entgegengesetzte Richtung geschwenkt ist (Kopftieflage). Die Zahnräder 15 befinden sich dabei am anderen Ende der Zahnstangen 14 verglichen mit der in Fig. 4 dargestellten Position. Um von der in Fig. 4 gezeigten Position in die in Fig. 5 gezeigte Position zu gelangen, wird das Tischgestell 2 mittels der Zahnräder 15 und der Zahnstangen 14 verschoben. Die Führungsstangen 9 bewegen sich dabei in zur Zeichenebene parallelen Ebenen. Der Gelenkpunkt B einer Führungsstange 9 bewegt sich dabei anfangs entgegen dem Uhrzeigersinn um die Achse 12 solange, bis der Tisch 1 in der horizontalen Lage angelangt ist. Danach bewegt sich dieser Gelenkpunkt B im Uhrzeigersinn um die Achse 12, bis die Führungsstange 9 in der in Fig. 5 dargestellten Position angelangt ist.

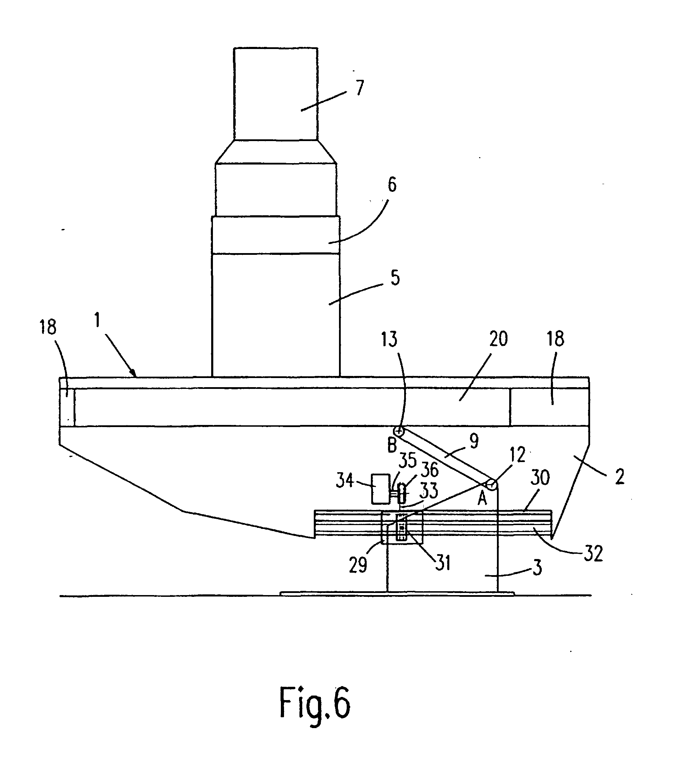

In Fig. 6 ist eine alternative Ausführungsform eines erfindungsgemäßen Röntgengeräts mit einem Spindelantrieb dargestellt. An der Basiseinheit 3 ist ein Gleitklotz 29 gelenkig angebracht, der in einer am Tischgestell 2 angebrachten Führungsschiene 30 gleitet und der Schwenkbewegung des Tischgestells 2 folgen kann. In dem Gleitklotz 30 sitzt eine Gewindemutter 31, die auf einer fest am Tischgestell 2 unterhalb der Führungsschiene 30 angebrachten Spindel 32 mit einem Gewinde sitzt, welches zu dem Gewinde der Gewindemutter 31 paßt. Die Gewindemutter 31 wird beispielsweise über eine Kette 33 von einer am Tischgestell 2 angebrachten und von einem Motor 34 angetriebenen Welle 35 mit einem Zahnrad 36 angetrieben. Wenn sich die Gewindemutter 31 dreht, wird das Tischgestell 2 mit der Spindel 32 gegenüber der Basiseinheit 3 verschoben, da die Gewindemutter 31 zusammen mit dem Gleitklotz 30 an der Basiseinheit 3 befestigt ist und nur der Schwenkbewegung des Tischgestells 2 folgen kann, nicht aber eine Verschiebung in horizontaler Richtung machen kann. Ein Großteil des Gewichts lastet bei dieser Ausgestaltung ebenfalls auf dem Gleitklotz und der Führungsschiene und nicht auf der Gewindemutter und der Spindel, um den Verschleiß an diesen Bauteilen gering zu halten.

Die Führungsstangen 9 müssen so ausgestaltet sein, daß sie Zug- und Druckkräfte aufnehmen können, die in verschiedenen Positionen des Tischgestells 2 verschieden groß sind. Die Lage der Achsen 11, 12 und 13 ist so ausgewählt, daß sich einerseits eine minimale Tischhöhe ergibt, wobei gleichzeitig eine ausreichende Fußfreiheit unterhalb des Tischgestells 2 für das Bedienpersonal bzw. den Patienten gewährleistet ist. Andererseits sollen die Kräfte auf die Lager möglichst gut verteilt sein, um den Verschleiß an Bauteilen möglichst gering zu halten.

Bei der in Fig. 1 gezeigten Ausführungsform mit einer Tischhöhe von etwa 850 mm sind die Gelenkpunkte A 435 mm und die Gelenkpunkte B 625 mm über dem Boden angeordnet. Die Achse 11 verläuft 255 mm über dem Boden. Der Abstand zwischen den Gelenkpunkten A und B beträgt etwa 400 mm und zwischen den Gelenkpunkten A und der Achse 11 etwa 370 mm.

Allgemein gilt, daß der am Tischgestell angebrachte Teil der Verschiebevorrichtung (z.B. in Fig. 1 die Zahnstange) nur auf einer Achse an der Basiseinheit gelagert ist, damit das Tischgestell neben der Verschiebebewegung auch eine Schwenkbewegung machen kann. Die Gelenkpunkte A dürfen nicht in der senkrecht zum Boden verlaufenden Ebene liegen, in der die Achse 11 liegt. Die Gelenkpunkte B sind derart angeordnet, daß ihre Höhe vom Boden größer ist als die Höhe der Gelenkpunkte A.

Bei der dargestellten Ausführung des Röntgendiagnostikgeräts sind jeweils ein Paar von Zahnrädern, Zahnstangen, Gleitklötzen, Führungsschienen und Führungsstangen vorhanden. Für die erfindungsgemäße Lösung der gestellten Aufgabe ist dies jedoch nicht erforderlich. Möglich wäre auch eine Ausgestaltung, bei der die genannten Elemente nur einzeln oder bei der nur zusammengehörige Elemente, z.B. Zahnrad und Zahnstange, jeweils doppelt vorgesehen ist.

Für die Erfindung spielt auch keine Rolle, daß das Tischgestell von einem in eine Zahnstange greifenden Zahnrad oder einem Spindelantrieb angetrieben wird. Der Antrieb könnte auch beispielsweise hydraulisch erfolgen. Wichtig ist lediglich, daß durch den Antrieb eine Verschiebung des Tischgestells gegenüber der Basiseinheit bewirkt wird.

Insgesamt ergibt sich bei einem erfindungsgemäßen Röntgendiagnostikgerät eine im gesamten Schwenkbereich von mindestens 120° eine gleichmäßig schnelle und ruhige Schwenkung des Tischgestells ohne ruckartige Bewegungen.

Das Schwenken des Tischgestells wird außerdem durch relativ einfache und in der Herstellung kostengünstige Bauteile bewirkt. Der Aufbau und die Zusammensetzung der Bauteile ist sehr einfach, weshalb das Gerät sehr zuverlässig und nahezu wartungsfrei arbeitet.

## Patentansprüche

1. Röntgendiagnostikgerät mit einem Tischgestell (2) und mit einer Basiseinheit (3) zur Lagerung des Tischgestells (2), welches um eine horizontale Achse schwenkbar ist, und mit einer Verschiebevorrichtung (4, 14, 15, 31, 32) zur Verschiebung des Tischgestells (2) gegenüber der Basiseinheit (3),
**dadurch gekennzeichnet, daß** an einem Gelenkpunkt (A) an der Basiseinheit (3) ein erster Punkt einer Führungsstange (9) angebracht ist und daß an einem Gelenkpunkt (B) am Tischgestell (2) ein zweiter Punkt der Führungsstange (9) angebracht ist, so daß bei der Verschiebung des Tischgestells (2) gegenüber der Basiseinheit (3) eine Schwenkbewegung des Tischgestells (2) erzwungen wird.

2. Röntgendiagnostikgerät nach Anspruch 1,
**dadurch gekennzeichnet, daß** das Tischgestell (2) eine Führungsschiene (8, 30) aufweist, die auf einem an der Basiseinheit (3) gelenkig angebrachten Gleitklotz (10, 29) gleitet.

3. Röntgendiagnostikgerät nach Anspruch 1 oder 2,
**dadurch gekennzeichnet, daß** das Tischgestell (2) zwei parallele Führungsschienen (8, 30) und die Basiseinheit (3) zwei gelenkig angebrachte Gleitklötze (10, 29) aufweist, auf denen jeweils eine der Führungsschienen (8, 30) gleitet.

4. Röntgendiagnostikgerät nach einem der Ansprüche 1 bis 3,
**dadurch gekennzeichnet, daß** zwei parallele Führungsstangen (9) vorgesehen sind und daß die Gelenkpunkte (A, B) an der Basiseinheit (3) bzw. am Tischgestell (2) jeweils auf einer gemeinsamen horizontalen Achse (12, 13) liegen.

5. Röntgendiagnostikgerät nach einem der Ansprüche 1 bis 5,
**dadurch gekennzeichnet, daß** die Verschiebevorrichtung (4, 14, 15, 31, 32) eine am Tischgestell (2) angebrachte Zahnstange (14) und ein an der Basiseinheit (3) drehbar gelagertes, in die Zahnstange (14) greifendes Zahnrad (15) umfaßt.

6. Röntgendiagnostikgerät nach einem der Ansprüche 1 bis 4,
**dadurch gekennzeichnet, daß** die Verschiebevorrichtung (4, 14, 15, 31, 32) zwei parallele, am Tischgestell (2) angebrachte Zahnstangen (14) und zwei parallele, an der Basiseinheit (3) drehbar gelagerte, in jeweils eine Zahnstange (14) greifende Zahnräder (15) umfaßt, welche auf einer gemeinsamen, von einem Motor (4) angetriebenen Antriebswelle (19) befestigt sind.

7. Röntgendiagnostikgerät nach einem der Ansprüche 1 bis 4,
**dadurch gekennzeichnet, daß** die Verschiebevorrichtung (4, 14, 15, 31, 32) eine am Tischgestell (2) angebrachte Spindel (32) und eine an der Basiseinheit (3) drehbar gelagerte, in die Spindel (32) greifende Gewindemutter (31) umfaßt.

## Claims

1. An X-ray diagnostic apparatus, including a table underframe (2) and a base unit (3) for supporting the table underframe (2) which can be swiveled about a horizontal axis, and also including a displacement device (4, 14, 15, 31, 32) for displacing the table underframe (2) relative to the base unit (3),
**characterized in that** a first point of a guide rod (9) is arranged at a hinge point (A) on the base unit (3) and a second point of the guide rod (9) is arranged at a hinge point (B) on the table underframe (2), so that a swiveling motion of the table underframe (2) is imposed upon displacement of the table underframe (2) relative to the base unit (3).

2. An X-ray diagnostic apparatus as claimed in Claim 1,
**characterized in that** the table underframe (2) is provided with a guide rail (8, 30) which slides on a slide block (10, 29) which is hinged to the base unit (3).

3. An X-ray diagnostic apparatus as claimed in Claim 1 or 2,
**characterized in that** the table underframe (2) is provided with two parallel guide rails (8, 30) and the base unit (3) is provided with two hinged slide blocks (10, 29) on which a respective one of the guide rails (8, 30) slides.

4. An X-ray diagnostic apparatus as claimed in any one of the Claims 1 to 3,
**characterized in that** there are provided two parallel guide rods (9), the hinge points (A, B) on the base unit (3) and on the table underframe (2), respectively, always being situated on a common horizontal shaft (12, 13).

5. An X-ray diagnostic apparatus as claimed in any one of the Claims 1 to 4,
**characterized in that** the displacement device (4, 14, 15, 31, 32) includes a toothed rack (14) which is mounted on the table underframe (2) and a gearwheel (15) which is rotatably journalled on the base unit (3) and engages the toothed rack (14).

6. An X-ray diagnostic apparatus as claimed in any one of the Claims 1 to 4,
**characterized in that** the displacement device (4, 14, 15, 31, 32) includes two parallel toothed racks (14) which are mounted on the table underframe (2) and two parallel gearwheels (15) which are rotatably journalled on the base unit (3) and each of which engages a respective toothed rack (14), said gearwheels being mounted on a common drive shaft (19) which is driven by a motor (4).

7. An X-ray diagnostic apparatus as claimed in any one of the Claims 1 to 4,
**characterized in that** the displacement device (4, 14, 15, 31, 32) includes a spindle (32) which is mounted on the table underframe (2) and a threaded nut (31) which is rotatably joumalled on the base unit (3) and engages the spindle (32).

## Revendications

1. Appareil de diagnostic radiographique avec un châssis de table (2) et avec une unité de base (3) pour le logement du châssis de table (2) qui peut pivoter autour d'un axe horizontal et avec un dispositif de coulissement (4, 14, 15, 31, 32) pour le coulissement du châssis de table (2) par rapport à l'unité de base (3),
**caractérisé en ce qu'**un premier point d'une tige de guidage (9) est appliqué contre un point d'articulation (A) sur l'unité de base (3) et qu'un deuxième point de la tige de guidage (9) est appliqué contre un point d'articulation (B) sur le châssis de table de telle sorte qu'un mouvement pivotant du châssis de table (2) soit forcé en cas de glissement du châssis de table (2) par rapport à l'unité de base (3).

2. Appareil de diagnostic radiographique selon la revendication 1,
**caractérisé en ce que** le châssis de table (2) présente un rail de guidage (8, 30) qui glisse sur un glisseur (10, 29) articulé sur l'unité de base (3).

3. Appareil de diagnostic radiographique selon l'une des revendications 1 ou 2,
**caractérisé en ce que** le châssis de table (2) présente deux rails de guidage (8, 30) parallèles et l'unité de base (3) deux glisseurs (10, 29) articulés sur lesquels glissent respectivement l'un des rails de guidage (8, 30).

4. Appareil de diagnostic radiographique selon l'une des revendications 1 à 3,
**caractérisé en ce qu'**il est prévu deux tiges de guidage (9) parallèles et que les points d'articulation (A, B) sur l'unité de base (3) ou le châssis de table (2) se trouvent respectivement sur un axe horizontal commun (12, 13).

5. Appareil de diagnostic radiographique selon l'une des revendications 1 à 5,
**caractérisé en ce que** le dispositif de glissement (4, 14, 15, 31, 32) comprend une crémaillère (14) logée sur le châssis de table (2) et une roue dentée (15) logée à rotation sur l'unité de base (3) et entrant en prise dans la crémaillère (14).

6. Appareil de diagnostic radiographique selon l'une des revendications 1 à 4,
**caractérisé en ce que** le dispositif de coulissement (4, 14, 15, 31, 32) comprend deux crémaillères (14) parallèles logées sur le châssis de table (2) et deux roues dentées (15) parallèles logées à rotation sur l'unité de base (3) et entrant en prise dans une crémaillère (14) respective, qui sont fixées sur un arbre d'entraînement (19) commun entraîné par un moteur (4).

7. Appareil de diagnostic radiographique selon l'une des revendications 1 à 4,
**caractérisé en ce que** le dispositif de coulissement (4, 14, 15, 31, 32) comprend un axe (32) logé sur le châssis de table (2) et un écrou fileté (31) entrant en prise dans l'axe (32) et logé à rotation sur l'unité de base (3).
